# EUROPEAN PATENT SPECIFICATION

(11) **EP 4 192 536 B1**
(45) Date of publication and mention of the grant of the patent: **09.10.2024**
(21) Application number: 21762796.7
(22) Date of filing: 04.08.2021
(51) Int. Cl.: A61L 27/16, A61L 27/56

(54) **HIERARCHICAL SCAFFOLDS FOR THE REGENERATION AND/OR SIMULATION AND/OR REPLACEMENT OF BONE TISSUE**
HIERARCHISCHE GERÜSTE ZUR REGENERATION UND/ODER SIMULATION UND/ODER ZUM ERSATZ VON KNOCHENGEWEBE
ÉCHAFAUDAGES HIÉRARCHIQUES POUR LA RÉGÉNÉRATION ET/OU LA SIMULATION ET/OU LE REMPLACEMENT DE TISSU OSSEUX

(30) Priority: 07.08.2020 IT 202000019621
(43) Date of publication of application: 14.06.2023
(73) Proprietor: Alma Mater Studiorum - Università di Bologna, 40126 Bologna (IT)
(72) Inventor: SENSINI, Alberto, 40131 Bologna (IT); ZUCCHELLI, Andrea, 40136 Bologna (IT); CRISTOFOLINI, Luca, 40136 Bologna (IT); GUALANDI, Chiara, 40126 Bologna (IT); FOCARETE, Maria Letizia, 40126 Bologna (IT)
(74) Representative: Di Giovine, Paolo
(86) International application number: PCT/IB2021/057133
(87) International publication number: WO 2022/029641

(56) References cited:
- WO-A1-2010/009320
- WO-A1-2016/077551
- WO-A1-2018/229615
- WO-A2-2010/096469
- US-A1- 2014 207 248

## Description

### FIELD OF THE INVENTION

The present invention relates to a process for the implementation of a porous hierarchical scaffold characterized by internal pores organized according to a hierarchical structure, for the regeneration, replacement and/or simulation of a bone tissue, in particular of the trabecular and/or cortical bone tissue. The present invention further relates to the porous hierarchical scaffold obtainable by such process and uses thereof. The invention further relates to the use of a sacrificial hierarchical scaffold for the preparation of such porous hierarchical scaffold.

### STATE OF ART

The use of scaffolds, or biomimetic three-dimensional supports made of synthetic or natural material, represents a strategy of proved effectiveness in the field of tissue engineering for the regeneration or replacement of damaged tissues.

A scaffold typically is devised to perform the following functions of: (i) promoting the cell-biomaterial interaction, the cell adhesion and the cell proliferation; (ii) allowing the transportation of gases and nutrients; (iii) if bioabsorbable, biodegrading at a speed approaching the tissue regeneration rate under the culture conditions of interest; (iv) causing a minimum degree of inflammation or *in vivo* toxicity; (v) having mechanical properties similar to the tissue which one wishes to reconstruct.

In particular, a scaffold devised for the repair or replacement of a bone tissue is required to have, apart from the above-mentioned base requirements, specific morphological features, essential to sustain the regeneration of the bone tissue in physiological environment. Among the morphological requirements of greater relevance, high internal porosity stands out, typical of the connective tissues.

The dimensional scales, the interconnecting level and the spatial distribution of the pores within a bone tissue strictly depend upon the organization of the extracellular matrix. The trabecular bone tissue, for example, consists of trabeculae made of collagen and hydroxyapatite, which assume an interconnected structure characterized by high porosity.

The cortical bone surrounds the trabecular bone, and it is characterized by a compact structure consisting of concentric circular (osteons) lamellar structures (lamellae), in the central portion thereof an axial canal is located, known as Haversian canal, inside thereof a blood vessel is placed. Haversian canals, in turn, are interconnected to each other through Volkmann's canals. Among the various lamellae composing osteons, moreover, there are additional and interconnected pores, having smaller sizes than those of Haversian canals, called lacunae, inside thereof osteocytes are placed and communicate between each other. In the external portion of the bone, instead, there is a fibrous membrane called periosteum, consisting of collagen fibers, having the function of protection, nutrition and interconnection with the surrounding soft tissues.

The bones of human and/or animal body often differ greatly between each other for shape and existing percentages of trabecular and/or cortical tissue. In the bones known as "long bones", for example, the areas richer in trabecular bone are those at the epiphysis (ends) where the cortical bone is present only in a thin layer of coating. In the central area of the long bones, instead, called diaphysis, the bone has a central cavity, inside thereof the bone marrow is housed, surrounded by a thin layer of cortical bone, where the trabecular one, on the contrary, is absent.

An ideal scaffold as bone substitute must have internal pores having sizes and degree of interconnection similar to the bone tissue which one wishes to reconstruct, so as to favour the mechanisms of adhesion, proliferation and colonization of the whole support by the cells, as well as the generation of a vascular network which is capable of sustaining the formation and *in vivo* maturation of the new bone.

The capability of reproducing as faithfully as possible the degree of interconnection and the complicated hierarchical structure characterizing the trabecular or cortical bone tissues, then represents a fundamental parameter in designing bone substitutes, and up to date it is one of the most complex challenges in the tissue engineering field.

The International patent application WO2018229615 describes multiscale hierarchical scaffold for the regeneration, replacement, simulation of muscular and nervous tissue.

WO 2010/096469 A2 discloses a method of forming a bone scaffold comprising electrospinning fibers, filling the space between the fibers with a polymeric material to form a matrix and removing the fibers.

From this a strong need is found for developing new processes for the implementation of multiscale porous hierarchical scaffolds which are capable of miming in a faithful and accurate way the hierarchical structure typical of the bone tissue, so as to support the formation of the three-dimensional tissue.

### SUMMARY OF THE INVENTION

The scope of the invention is defined by the claims. Any references in the description to methods of treatment refer to the products of the present invention for use in a method for treatment.

The object of the present invention is to provide a scaffold for the replacement, reconstruction and/or simulation of the bone tissue, through the implementation of a porous construct with hierarchical and multiscale three-dimensional structure (porous hierarchical scaffold), capable of reproducing as faithfully as possible the morphological, physical and mechanical features typical of the connective tissues such as bone.

In the present description under porous hierarchical support and/or scaffold any three-dimensional anisotropic device or construct is meant, comprising pores of different dimensional scales (from nanometric to micrometric and millimetric scale), interconnected between each other and/or organized according to a hierarchical order to mime as faithfully as possible the extracellular matrix of the bone tissue.

The authors of the present invention have succeeded in developing hierarchical scaffolds having internal pores capable of miming perfectly, individually or contemporarily, the high degree of porosity and interconnection of the trabecular and/or cortical bone tissue.

In particular, the porous hierarchical scaffold, the invention relates to, is implemented by integrating a sacrificial hierarchical structure or scaffold consisting of nanofibers, obtained by electrospinning, inside a matrix. At the end of the integration procedure, the process devised by the inventors provides for the removal of the sacrificial hierarchical scaffold, for example by dissolution of the sacrificial component in a suitable process, by generating, inside the solid matrix, controlled pores corresponding to the removed nanofibers, wholly similar to those of the extracellular matrix of the bone tissue.

According to an aspect of the invention, the sacrificial structure or scaffold used for the production of the porous hierarchical scaffold can include a plurality of sacrificial clusters of nanofibers, joined and compacted to each other by an electrospun porous sheath of nanofibers.

Such sacrificial hierarchical scaffold in case can consist of several sub-groups of clusters of electrospun nanofibers, in turn joined between each other in an additional hierarchical level by an additional electrospun sheath produced similarly to the preceding one.

By varying the arrangement of the groups and/or the clusters of electrospun nanofibers inside the sacrificial hierarchical structure, it is possible, after removing the same, to produce a porous hierarchical scaffold capable of reproducing faithfully the hierarchical and multiscale organization characteristic of the bone tissue.

The invention then provides a flexible process, adaptable to the implementation of biomimetic porous hierarchical scaffolds which can be integrated inside and/or simulate the *in vitro* operation of any body bone segment.

The high porosity and the high degree of interconnection typical of the trabecular bone tissue are perfectly mimed by the porous hierarchical scaffold the invention relates to: by using sacrificial clusters of nanofibers, in turn axially aligned according to the axis of the scaffold and/or arranged randomly, it is possible to simulate the aggregation level of the trabecular bone.

Analogously, by using axially aligned clusters of electrospun nanofibers and by varying the sizes thereof, it is possible to simulate the porosity and the degree of interconnection of Haversian canals, Volkmann's canals, or of lacunae characteristic of the cortical bone tissue.

Moreover, by joining between each other sub-groups of clusters of nanofibers with electrospun porous sheaths, it is possible to simulate endosteum and/or the cementing lines which form between osteons. The porous hierarchical scaffold implementable by means of the process, the invention relates to, can even have a nanofibrous external membrane made of polymeric material, obtained by electrospinning, capable of miming perfectly the structure and function of the periosteum coating the human bone, on one side guaranteeing protection to the underlying porous hierarchical scaffold and at the same time allowing the passage of the cells therethrough so as to reconstruct the extracellular matrix of the bone tissue.

The capability of reproducing faithfully the morphological features and all aggregation levels of the bone tissue, from the nanoscopic order of magnitude to the macroscopic one, in case even within the same scaffold, results to be of fundamental importance for the implementation of a bone substitute.

The porous hierarchical scaffold obtained by means of the process the invention relates to, independently from the material thereof it consists, can be implanted in the human and/or animal body by reproducing the morphological and structural features of the natural bone and by allowing the cells to filtrate between the pores and sustain the reconstruction of the natural bone matrix.

The herein described hierarchical scaffold can advantageously be used in different applications, for example in the biomedical field, in orthopaedic or veterinary field as implantable prosthetic device, by favouring the cell proliferation and the tissue regeneration. A porous hierarchical scaffold made of bioabsorbable material, in particular, can favour colonization and proliferation of osteocytes, by sustaining the degradation process of the implant until complete regeneration of the patient's natural bone.

In its biostable version, instead, such porous scaffold can be used for the simulation of the *in vitro* bone tissue and/or to act as connection or reinforcement in prosthetic or robotic applications. When prepared in synthetic material, alternatively it can be applied in the production of sensors, in the production of actuators and guides for flexible robotics or in the production of synthetic bones for the simulation of *in vitro* surgical interventions or for mechanical tests.

A first aspect of the present invention then relates to a process for the preparation of a porous hierarchical scaffold for the replacement, repair, regeneration, reconstruction and/or simulation of a bone tissue, in particular of the trabecular and/or cortical bone tissue comprising the following steps:
i) preparing by electrospinning a plurality of sacrificial clusters of nanofibers;
ii) positioning said plurality of clusters prepared in step i) so as to form at least a single group;
iii) electrospinning nanofibers so as to coat said at least one group of clusters with a sacrificial porous sheath, consisting of nanofibers, so as to form an external coating and to compact the plurality of clusters prepared with step i) ;
iv) inserting the sacrificial hierarchical scaffold obtained with step iii) inside a hollow container, leaving the terminal ends exposed;
v) filling said hollow container with a material capable of permeating within and/or incorporating said sacrificial hierarchical scaffold;
vi) allowing said material to solidify;
vii) removing said sacrificial hierarchical scaffold so as to produce said porous hierarchical scaffold having internal pores organized according to a hierarchical structure.

### BRIEF DESCRIPTION OF FIGURES

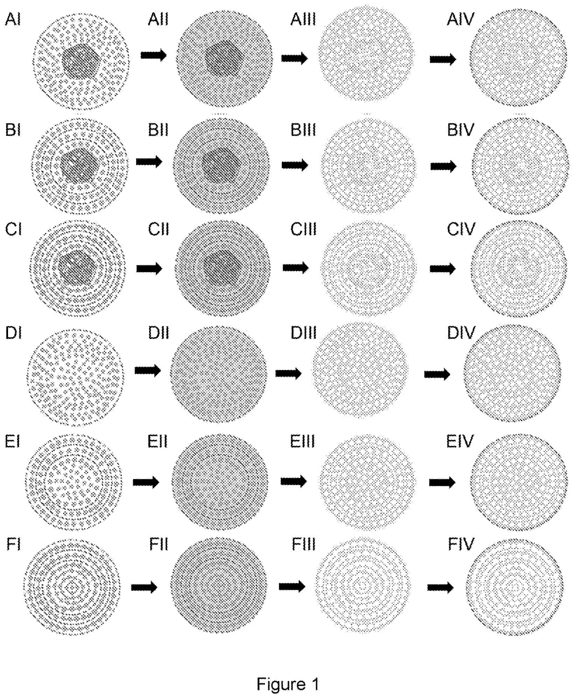

Figure 1 - Schematic representation, seen from top, of embodiments of the sacrificial hierarchical scaffold and the complete porous hierarchical scaffold in the various process steps.
   AI) Sacrificial electrospun hierarchical scaffold consisting of a plurality of clusters of nanofibers, having in the centre a group of clusters of nanofibers arranged randomly and/or broken-up clusters of nanofibers arranged randomly, coated with an external sheath of nanofibers. AII) The sacrificial electrospun hierarchical scaffold is incorporated in a matrix. AIII) The sacrificial electrospun hierarchical scaffold is dissolved and/or removed with an adequate solvent and/or process by leaving in the matrix corresponding cavities. AIV) Possible electrospinning of a membrane of nanofibers external to the scaffold.
   BI) Sacrificial electrospun hierarchical scaffold consisting of a plurality of clusters of nanofibers kept together by concentric electrospun sheaths, having in the centre a group of clusters of nanofibers arranged randomly and/or broken-up clusters of nanofibers arranged randomly, coated with an external sheath of nanofibers. BII) The sacrificial electrospun hierarchical scaffold is incorporated in a matrix. BIII) The sacrificial electrospun hierarchical scaffold is dissolved and/or removed with an adequate solvent and/or process by leaving in the matrix corresponding cavities. BIV) Possible electrospinning of a membrane of nanofibers external to the scaffold.
   CI) Sacrificial electrospun hierarchical scaffold consisting of a plurality of clusters of nanofibers crossed by groups of circumferential (and/or in case radial) clusters of nanofibers kept together by concentric sheaths, having in the centre a group of clusters of nanofibers arranged randomly and/or broken-up clusters of nanofibers arranged randomly, coated with an external sheath of nanofibers. CII) The sacrificial electrospun hierarchical scaffold is incorporated in a matrix. CIII) The sacrificial electrospun hierarchical scaffold is dissolved and/or removed with an adequate solvent and/or process by leaving in the matrix corresponding cavities. CIV) Possible electrospinning of a membrane of nanofibers external to the scaffold.
   DI) Sacrificial electrospun hierarchical scaffold consisting of a plurality of clusters of nanofibers, coated with an external sheath of nanofibers. DII) The sacrificial electrospun hierarchical scaffold is incorporated in a matrix. DIII) The sacrificial electrospun hierarchical scaffold is dissolved and/or removed with an adequate solvent and/or process by leaving in the matrix corresponding cavities. DIV) Possible electrospinning of a membrane of nanofibers external to the scaffold.
   EI) Sacrificial electrospun hierarchical scaffold consisting of a plurality of clusters of nanofibers kept together by concentric electrospun sheaths, coated with an external sheath of nanofibers. EII) The sacrificial electrospun hierarchical scaffold is incorporated in a matrix. EIII) The sacrificial electrospun hierarchical scaffold is dissolved and/or removed with an adequate solvent and/or process by leaving in the matrix corresponding cavities. EIV) Possible electrospinning of a membrane of nanofibers external to the scaffold.
   FI) Sacrificial electrospun hierarchical scaffold consisting of a plurality of clusters of nanofibers crossed by groups of circumferential (and/or in case radial) clusters of nanofibers kept together by concentric electrospun sheaths, coated with an external sheath of nanofibers. FII) The sacrificial electrospun hierarchical scaffold is incorporated in a matrix. FIII) The sacrificial electrospun hierarchical scaffold is dissolved and/or removed with an adequate solvent and/or process by leaving in the matrix corresponding cavities. FIV) Possible electrospinning of a membrane of nanofibers external to the scaffold.

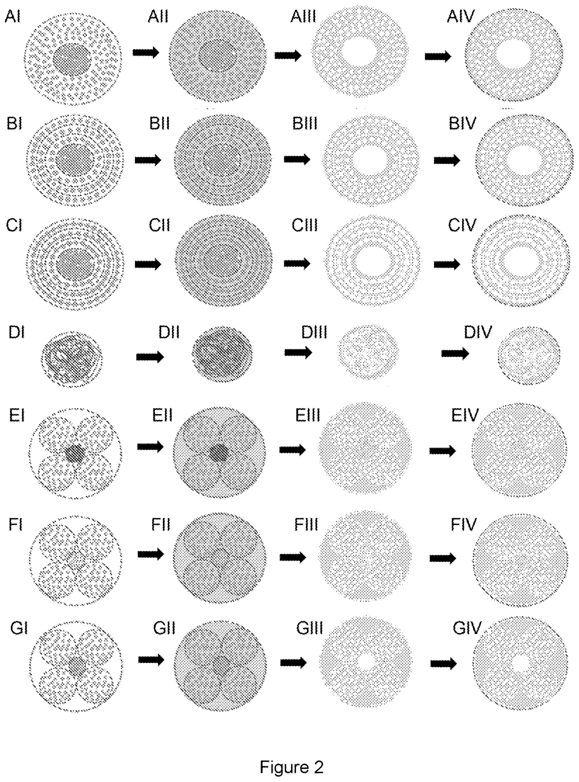

Figure 2 - Schematic representation, seen from top, of embodiments of the sacrificial hierarchical scaffold and of the complete porous hierarchical scaffold, during the various process steps.
   AI) Sacrificial electrospun hierarchical scaffold consisting of a plurality of clusters of nanofibers coated with an external sheath of nanofibers, having in the centre an electrospun hierarchical structure consisting of very compact clusters of nanofibers and kept together by an electrospun sheath. AII) The sacrificial electrospun hierarchical scaffold is incorporated in a matrix. AIII) The sacrificial electrospun hierarchical scaffold is dissolved and/or removed with an adequate solvent and/or process by leaving in the matrix corresponding cavities, in particular by leaving an axial macroscopic cavity in the centre of the scaffold. AIV) Possible electrospinning of a membrane of nanofibers external to the scaffold.
   BI) Sacrificial electrospun hierarchical scaffold consisting of a plurality of clusters of nanofibers kept together by concentric electrospun sheaths, having in the centre a sacrificial electrospun hierarchical scaffold consisting of very compact axial clusters of nanofibers and kept together by an electrospun sheath. BII) The sacrificial electrospun hierarchical scaffold is incorporated in a matrix. BIII) The sacrificial electrospun hierarchical scaffold is dissolved and/or removed with an adequate solvent and/or process by leaving in the matrix corresponding cavities, in particular by leaving an axial macroscopic cavity in the centre of the scaffold. BIV) Possible electrospinning of a membrane of nanofibers external to the scaffold.
   CI) Sacrificial electrospun hierarchical scaffold consisting of a plurality of axially aligned clusters of nanofibers crossed by groups of circumferential (and/or in case radial) clusters of nanofibers kept together by concentric electrospun sheaths, having in the centre a sacrificial hierarchical scaffold consisting of very compact axial clusters of nanofibers and kept together by an electrospun sheath. CII) The sacrificial electrospun hierarchical scaffold is incorporated in a matrix. CIII) The sacrificial electrospun hierarchical scaffold is dissolved and/or removed with an adequate solvent and/or process by leaving in the matrix corresponding cavities, in particular by leaving an axial macroscopic cavity in the centre of the scaffold. CIV) Possible electrospinning of a membrane of nanofibers external to the scaffold.
   DI) Sacrificial electrospun hierarchical scaffold consisting of a group of clusters of nanofibers arranged randomly and/or broken-up clusters of nanofibers arranged randomly, coated with an external sheath of nanofibers. DII) The sacrificial electrospun hierarchical scaffold is incorporated in a matrix. DIII) The sacrificial electrospun hierarchical scaffold is dissolved and/or removed with an adequate solvent and/or process by leaving in the matrix corresponding cavities. DIV) Possible electrospinning of a membrane of nanofibers external to the scaffold.
   EI) Plurality of sacrificial electrospun hierarchical scaffolds consisting of a plurality of clusters of nanofibers coated with external sheaths of nanofibers, having in the centre a sacrificial electrospun hierarchical scaffold consisting of a group of clusters of nanofibers arranged randomly and/or broken-up clusters of nanofibers arranged randomly, coated with an external sheath of nanofibers. EII) The sacrificial electrospun hierarchical scaffold is incorporated in a matrix. EIII) The sacrificial electrospun hierarchical scaffold is dissolved and/or removed with an adequate solvent and/or process by leaving in the matrix corresponding cavities. EIV) Possible electrospinning of a membrane of nanofibers external to the scaffold.
   FI) Plurality of sacrificial electrospun hierarchical scaffolds consisting of a plurality of nanofibers coated with external sheaths of nanofibers, coated with an external sheath of nanofibers. FII) The sacrificial electrospun hierarchical scaffold is incorporated in a matrix. FIII) The sacrificial electrospun hierarchical scaffold is dissolved and/or removed with an adequate solvent and/or process by leaving in the matrix corresponding cavities. FIV) Possible electrospinning of a membrane of nanofibers external to the scaffold.
   GI) Plurality of sacrificial electrospun hierarchical scaffolds consisting of a plurality of clusters of nanofibers coated with external sheaths of nanofibers, having in the centre a sacrificial electrospun hierarchical scaffold consisting of very compact axial clusters of nanofibers and kept together by an electrospun sheath. GII) The sacrificial electrospun hierarchical scaffold is incorporated in a matrix. GIII) The sacrificial electrospun hierarchical scaffold is dissolved and/or removed with an adequate solvent and/or process by leaving in the matrix corresponding cavities, in particular by leaving an axial macroscopic cavity in the centre of the scaffold. GIV) Possible electrospinning of a membrane of nanofibers external to the scaffold.

### DETAILED DESCRIPTION OF THE INVENTION

The present invention relates to a porous hierarchical scaffold, the processes for the production the production thereof and uses thereof.

In the present description under "support or porous hierarchical scaffold" one means: each device, structure or anisotropic three-dimensional construct characterized by pores with different dimensional scales (from nanometric to micrometric and millimetric scale), which are interconnected between each other according to a hierarchical order to reproduce as faithfully as possible the extracellular matrix of the tissue which one wants to reconstruct in its native state.

In the present description under "clusters of nanofibers" an electrospun structure is meant, having variable extension and/or section, consisting of nanofibers arranging with any orientation along the larger development direction of these constructs.

In the present description under "clusters of nanofibers with axial alignment (bundle)" an electrospun structure is meant, having variable extension and/or section, consisting of nanofibers which arrange with an alignment degree according to the axis of the bundle itself, that is along the along the larger development direction of these constructs.

In the present description under "clusters of twisted nanofibers (yarn)" an electrospun structure is meant, having variable extension and/or section, consisting of nanofibers arranging with a twisting degree according to the axis of the yarn itself, that is along the larger development direction of these constructs.

In the present description under "clusters of random nanofibers" an electrospun structure is meant, having variable extension and/or section, consisting of nanofibers which arrange randomly along the axis of the cluster itself, or along the larger development direction of these constructs.

In the present description under "ring-shaped cluster of nanofibers (ring bundle)" a ring-shaped electrospun structure is meant, with variable extension and section, consisting of nanofibers, with an alignment degree according to the axis of the ring-shaped cluster or ring bundle itself. Such construct configures morphologically like a closed ring, consisting of nanofibers arranged randomly and/or with an alignment degree along the axial direction of the ring.

Under the term "sacrificial" the present invention relates to a device, structure, scaffold or construct intended to be removed to allow the production of pores with controlled dimensional scales, organized according to a hierarchical order within a solid matrix, wholly similar to the typical pores of the bone tissue.

In particular, a "sacrificial" hierarchical scaffold according to the present invention, is an anisotropic three-dimensional electrospun construct, made of materials assembled at morphological level at different levels of dimensional scale, which acts as "mould" or "template", and it is intended to be removed, to allow the formation or controlled pores capable of miming perfectly the characteristic porosity of the bone extracellular matrix. An example of device, structure, "sacrificial" hierarchical construct or scaffold according to the present invention is a device, structure, electrospun construct or scaffold consisting of a material capable of being dissolved and/or removed by an adequate solvent and/or process, in turn not capable to dissolve and/or remove the matrix inside thereof said construct was incorporated and/or integrated. The removal of a similar device, structure, construct or sacrificial scaffold can be performed by dipping said construct inside a suitable solvent for the time required to the complete solubilization of the material thereof it consists.

In the present description under the expression "to form one single group" one means to form a set or cluster of several clusters, wherein each cluster in turn consists of nanofibers.

As already mentioned, the present invention relates to a process for the preparation of a porous hierarchical scaffold for the replacement, repair, regeneration, reconstruction and/or simulation of a bone tissue. Said process provides for the implementation of a sacrificial hierarchical scaffold, by electrospinning, consisting of one or more groups of clusters of nanofibers assembled according to a hierarchical organization, in turn kept together through one or more electrospun porous sheaths. Said sacrificial hierarchical scaffold can be incorporated and/or integrated inside a solid matrix and subsequently removed to allow the formation of controlled pores, having different dimensional scales and/or different degree of interconnection, inside said matrix, so as to form a porous hierarchical scaffold capable of reproducing faithfully the morphological and structural features of the wished bone tissue.

A sacrificial hierarchical scaffold which can be used in the process according to the present invention for example comprises a plurality of sacrificial clusters obtained by electrospinning consisting of nanofibers, arranged so as to form at least a single group of clusters.

The sacrificial hierarchical scaffold used for the preparation of a porous hierarchical scaffold according to the process, the invention relates to, further comprises at least a sacrificial sheath, obtained by electrospinning, which coats externally and compacts said plurality of clusters of nanofibers. Said sheath in turn consists of nanofibers and it is characterized by a porosity which is produced by overlapping layers of continuous fibers. Therefore, the porosity of said sacrificial sheath is interconnected, in other terms the pores of the sheath put into communication the external layer of the sheath of nanofibers with the innermost layer (in contact with the clusters and/or with an in case additional sheath and/or sheaths). The interconnection between the pores of the porous sheath will allow the material of the matrix to filter between the nanofibers by incorporating the sacrificial hierarchical scaffold.

Said sacrificial sheath of nanofibers then will allow to reproduce, after removal of the sacrificial hierarchical scaffold, the characteristic pores of the lacunae and of the canaliculi interconnecting osteons inside the cortical tissue, and/or osteons and the trabecular bone.

Therefore, a first aspect of the present invention relates to a process for the preparation of a porous hierarchical scaffold for the replacement, repair, regeneration reconstruction and/or simulation of a bone tissue, in particular of the trabecular and/or cortical bone tissue comprising the following steps:
i) preparing by electrospinning a plurality of sacrificial clusters of nanofibers;
ii) positioning said plurality of clusters prepared in step i) so as to form at least a single group;
iii) electrospinning nanofibers so as to coat said at least one group of clusters with a sacrificial porous sheath, consisting of nanofibers, so as to form an external coating and to compact the plurality of clusters prepared with step i) ;
iv) inserting the sacrificial hierarchical scaffold obtained with step iii) inside a hollow container, by leaving the terminal ends exposed;
v) filling said hollow container with a material capable of permeating within and/or incorporating said sacrificial hierarchical scaffold;
vi) allowing said material to solidify;
vii) removing said sacrificial hierarchical scaffold so as to obtain said porous hierarchical scaffold having internal pores organized according to a hierarchical structure.

According to an aspect of the present invention, the herein described process could further include, after step iii) and before step iv), the following steps:
- coating by electrospinning the sacrificial hierarchical scaffold obtained with step ii) with one or more layers each one comprising a plurality of sacrificial clusters of nanofibers;
- electrospinning nanofibers so as to coat said one or more layers of clusters each one with a sacrificial porous sheath, consisting of nanofibers, so as to form an external coating and to compact said plurality of clusters of nanofibers.

The just described steps will allow to obtain a sacrificial hierarchical scaffold characterized by the overlapping of several additional layers of single and/or interconnected clusters, wherein each one could be coated with a porous sheath consisting of nanofibers.

According to an embodiment, the process the invention relates to comprises the following steps:
i) preparing by electrospinning a plurality of sacrificial clusters of nanofibers;
ii) positioning said plurality of clusters prepared in step i) so as to form at least a single group;
iii) grasping said at least one group of clusters obtained in step ii) on a grip capable of rotating rigidly and in line thus by keeping said grasped group of clusters in a position suitable for the process of coating with sacrificial electrospun sheath;
iv) making a sacrificial sheath external to said at least one group of clusters grasped at step iii) by electrospinning, in particular by controlling the rotation parameters of the grasped group of clusters, the geometrical parameters of the setup, and process parameters;
v) inserting the sacrificial hierarchical scaffold obtained with step iv) inside a hollow container, by leaving the terminal ends exposed;
vi) filling said hollow container with a material capable of permeating within and/or incorporating said sacrificial hierarchical scaffold;
vii) allowing said material to solidify;
viii) removing said sacrificial hierarchical scaffold so as to obtain said porous hierarchical scaffold having internal pores organized according to a hierarchical structure.

According to an additional embodiment, the sacrificial hierarchical scaffold used for implementing the porous hierarchical scaffold the invention relates to, could advantageously comprise the joining of several sacrificial hierarchical scaffolds, that is the joining of a plurality of distinct groups of clusters of nanofibers, each group comprising an external sacrificial porous sheath capable of compacting said clusters, wherein said hierarchical structures and/or said plurality of distinct groups of clusters are kept together by an additional external sacrificial sheath, in turn obtained by electrospinning.

Therefore, an aspect of the invention relates to a process according to any one of the preceding embodiments, comprising the following steps:
(i) preparing by electrospinning a plurality of sacrificial clusters of nanofibers;
(ii) positioning said plurality of clusters prepared in step i) so as to form a plurality of groups of clusters;
(iii) grasping each group of clusters obtained in step ii) on a grip capable of rotating rigidly and in line thus by keeping each grasped group of clusters in a position suitable for the process of coating with sacrificial electrospun sheath;
(iv) making a sacrificial sheath external to each group of clusters grasped at step iii) by electrospinning, in particular by controlling the rotation parameters of the grasped group of clusters, the geometrical parameters of the setup, and process parameters;
(v) electrospinning nanofibers so as to coat said plurality of groups of clusters with a sacrificial porous sheath, consisting of nanofibers, so as to form an external coating and to compact said plurality of groups of clusters;
(vi) inserting the sacrificial hierarchical scaffold obtained with step v) inside a hollow container, by leaving the terminal ends exposed;
(vii) filling said hollow container with a material capable of permeating within and/or incorporating said sacrificial hierarchical scaffold;
(viii) allowing said material to solidify;
(ix) removing said sacrificial hierarchical scaffold so as to obtain said porous hierarchical scaffold having internal pores organized according to a hierarchical structure.

According to an aspect of the invention, the process according to one of the previously described embodiments can include an additional step of:
- electrospinning nanofibers so as to coat said porous hierarchical scaffold obtained in the final step with a membrane consisting of nanofibers, so as to form an external coating.

Said electrospun external membrane consisting of nanofibers can be made by fixing said porous hierarchical scaffold on a rotating machine by modulating adequately the spinning parameters. Such nanofibrous external membrane allows to reproduce the characteristic structure and the function of the periosteum coating the bone tissue.

According to an aspect of the invention said membrane consisting of nanofibers is prepared by electrospinning a solution of bioabsorbable or biostable and/or inert material of synthetic or natural origin. Examples of materials are polyesters, polyethers, polyurethanes, polyamides, polyolefins, polycarbonates, polyanhydrides, fluorinated polymers, acrylates, polysaccharides, proteins, polypeptides and copolymers and/or mixtures thereof.

In a preferred embodiment of the process the invention relates to, said membrane consisting of nanofibers is prepared by electrospinning a solution of polylactic acid and/or a mixture of polylactic acid and collagen.

According to an aspect of the invention the sacrificial clusters composing the sacrificial hierarchical scaffold, each one consisting of nanofibers, are positioned and/or connected to each other according to an axial alignment with respect to the sacrificial hierarchical scaffold, and/or according to a circumferential orientation with respect to the sacrificial hierarchical scaffold, and/or according to a radial orientation with respect to the sacrificial hierarchical scaffold, and/or randomly (random), and/or according to combinations thereof.

In an embodiment, for example, such clusters could be grouped to form a group of axially aligned clusters and/or clusters interconnected to each other with additional clusters, for example oriented circumferentially. Inside the group of clusters, clusters of nanofibers arranged randomly could be inserted.

In an alternative embodiment of the sacrificial hierarchical scaffold, one for example could advantageously use internal clusters of nanofibers compacted by the sheath and/or electrospun sheaths so as not to allow the material of the matrix to filter inside thereof. In this way, once removed the sacrificial electrospun hierarchical scaffold it will be possible to produce macroscopic and/or microscopic cavities capable to simulate the internal cavities of the bone inside thereof there is the marrow.

Therefore, in an embodiment of the present invention, said sacrificial clusters, each one consisting of nanofibers, could be positioned between each other according to an axial alignment with respect to the sacrificial hierarchical scaffold so as to form a central group of compact sacrificial clusters.

According to an embodiment, exemplified in Figure 1-AI, said sacrificial hierarchical scaffold consists, for example, of a plurality of clusters of nanofibers and of a central group of clusters of nanofibers arranged randomly and/or broken-up clusters of nanofibers arranged randomly, wherein said clusters are coated and/or kept together by an electrospun external porous sheath, consisting of nanofibers.

According to an embodiment, exemplified in Figure 1-BI, said sacrificial hierarchical scaffold consists of a plurality of clusters of nanofibers, and of a central group of clusters of nanofibers arranged randomly and/or of broken-up clusters of nanofibers arranged randomly, wherein said clusters are kept together by concentric internal porous sheaths and they are coated and/or kept together by an electrospun external porous sheath, consisting of nanofibers.

According to an embodiment, exemplified in Figure 1-CI, said sacrificial hierarchical scaffold consists of a plurality of clusters of nanofibers, crossed and/or connected by clusters of nanofibers arranged according to a circumferential (and/or in case radial) orientation, and by a central group of clusters of nanofibers arranged randomly and/or broken-up clusters of nanofibers arranged randomly. Said clusters are kept together by concentric internal porous sheaths and coated and/or kept together by an electrospun external porous sheath consisting of nanofibers.

According to an embodiment, exemplified in Figure 1-DI, said sacrificial hierarchical scaffold consists of a plurality of clusters of nanofibers, wherein said clusters are coated with an electrospun external porous sheath, consisting of nanofibers.

According to an embodiment, exemplified in Figure 1-EI, said sacrificial hierarchical scaffold consists of a plurality of clusters of nanofibers, wherein said clusters are kept together by concentric internal electrospun porous sheaths and they are coated and/or kept together by an electrospun external porous sheath, consisting of nanofibers. According to an embodiment, exemplified in Figure 1-FI said sacrificial hierarchical scaffold consists of a plurality of clusters of nanofibers, crossed and/or connected by clusters of nanofibers arranged according to a circumferential (and/or in case radial) orientation, wherein said clusters of nanofibers are kept together by concentric internal electrospun porous sheaths, and they are coated and/or kept together by an external electrospun porous sheath, consisting of nanofibers.

According to an embodiment, exemplified in Figure 2-AI, said sacrificial hierarchical scaffold consists of a plurality of clusters of nanofibers coated and/or kept together by an external electrospun porous sheath, consisting of nanofibers, and by a plurality of clusters of nanofibers to form a compact central group of clusters of nanofibers, which are kept together by an electrospun porous sheath of nanofibers.

According to an embodiment, exemplified in Figure 2-BI, said sacrificial hierarchical scaffold consists of a plurality of clusters of nanofibers, and of a plurality of clusters of nanofibers arranged according to an axial orientation to form a central group of compact clusters, wherein said clusters are kept together by concentric internal porous sheaths and they are coated and/or kept together by an electrospun external porous sheath, consisting of nanofibers.

According to an embodiment, exemplified in Figure 2-CI, said sacrificial hierarchical scaffold consists of a plurality of clusters of nanofibers, crossed and/or connected by clusters of nanofibers arranged according to a circumferential (and/or in case radial) orientation, and of a central group consisting of compact clusters of nanofibers arranged according to an axial alignment, wherein said clusters are kept together by concentric internal porous sheaths and they are coated and/or kept together by an external electrospun porous sheath, consisting of nanofibers.

According to an embodiment, exemplified in Figure 2-DI, said electrospun hierarchical scaffold consists of a group of clusters of nanofibers arranged randomly and/or of broken-up clusters of nanofibers arranged randomly, wherein said clusters are coated and/or kept together by an electrospun external porous sheath, consisting of nanofibers.

According to an embodiment, exemplified in Figure 2-EI, said sacrificial hierarchical scaffold consists of a plurality of groups consisting of clusters of nanofibers and by a central group of clusters of nanofibers arranged according to a random orientation and/or of broken-up clusters of nanofibers arranged according to a random orientation, wherein each group of clusters is coated and/or kept together by an electrospun porous sheath, and wherein said groups of clusters in turn are coated and/or kept together by an electrospun external porous sheath, consisting of nanofibers.

According to an embodiment, exemplified in Figure 2-FI, said sacrificial hierarchical scaffold consists of a plurality di groups consisting of clusters of nanofibers arranged according to an axial alignment each one coated and/or kept together by an electrospun porous sheath, wherein said groups of clusters in turn are coated and/or kept together by an electrospun external porous sheath, consisting of nanofibers.

According to an embodiment, exemplified in Figure 2-GI, said sacrificial hierarchical scaffold consists of a plurality of groups consisting of clusters of nanofibers, and of a central group of compact clusters of nanofibers, arranged according to an axial alignment, each group is coated and/or kept together by an electrospun porous sheath, and wherein said groups of clusters in turn are coated and/or kept together by an electrospun external porous sheath, consisting of nanofibers.

According to an aspect of the invention, the sacrificial clusters and said sacrificial porous sheath/s composing said sacrificial hierarchical scaffold consist of a dissolvable material, or a material capable of being solubilized and/or removed by a suitable solvent and/or process.

In particular, according to an aspect of the invention, said sacrificial clusters and said sacrificial porous sheath/s could be made of a material selected from polyesters, polyethers, polyurethanes, polyanhydrides, polycarbonates, polyamides, polyolefins, acrylates, fluorinated polymers and copolymers thereof, polysaccharides, proteins, polypeptides, and copolymers thereof and/or mixtures thereof.

In a preferred embodiment of the process the invention relates to, said sacrificial clusters and said sacrificial porous sheath/s are prepared by electrospinning a solution of pullulan and/or polyethylene oxide dissolved in suitable solvent, for example distilled water. The solution could be prepared for example with 5-30% (w/v) of pullulan in distilled water.

According to an embodiment, during the step of electrospinning nanofibers of pullulan of said clusters, an electric field could be applied with a voltage comprised between 1 and 100 kV, preferably 26 kV, by depositing the nanofibers on a collector rotating at high speed which allows an alignment level of the nanofibers.

According to an additional embodiment, the spinning conditions of the clusters of polyethylene oxide provide for the application of an electrical field with a voltage comprised between 1 and 100 kV, preferably 18 kV, by depositing the nanofibers on ca collector.

The nanofibers deposited on the collector can be subsequently collected together to form clusters of nanofibers and/or microfibers.

According to an embodiment, the electrospinning conditions on the rotating machine for the production of the sheath/s of nanofibers provide for the application of an electrical field with a voltage comprised between 1 and 100 kV, for an electrospinning period of time variable depending upon the thickness of the sheath to be obtained.

Advantageously, for the preparation of the sacrificial porous sheath/s the following process parameters will be applied:
- distance between the group of clusters and the collector less than 5 mm;
- rotation speed of the group of clusters between about 1-1000 rpm;
- periods of immobility of the group of clusters between about 1 sec-100 min;
- rotation periods of the group of clusters between 1 sec-100 min.

As mentioned, such process could be repeated by assembling additional layers of clusters grouped to form a group of single clusters and/or clusters interconnected to each other with additional clusters, for example oriented circumferentially, above the obtained structure and by reiterating the above-described process.

Similarly, by repeating the production process of the sacrificial sheath of nanofibers on a plurality di sacrificial hierarchical scaffolds, produced as previously described and joined together, it will be possible to obtain a sacrificial hierarchical scaffold in turn consisting of a plurality di sacrificial multiscale hierarchical scaffolds, all coated and compacted by an electrospun sheath of nanofibers.

In an embodiment of the nanofibers composing the sacrificial multiscale hierarchical scaffold and/or sheath and/or the clusters of nanofibers could be, from the morphology point of view, classical nanofibers consisting of one single phase (made of one single material and/or of a mixture of materials and/or loaded and/or functionalized materials) and/or nanofibers consisting of two or more phases (for example core-shell nanofibers, wherein under core-shell, nanofibers are meant made of different materials between central and external portion of the nanofiber itself) and/or hollow-shell nanofibers (wherein under hollow-shell, nanofibers are meant consisting of an internal central cavity along the axis of the nanofibers themselves) and/or porous nanofibers (under porous nanofibers, nanofibers are meant having pores along their surface and/or in their internal volume).

The average diameter of the nanofibers used for implementing the sacrificial hierarchical scaffold could be comprised between 10 and 10000 nm, in particular comprised between 100 and 1000 nm. The sacrificial hierarchical scaffold according to any one of the herein described embodiments could have a length comprised between 0.1 and 2000 mm, in particular between 0.5-200 mm, and an average diameter comprised between 1 and 200 mm, in particular between 0.1 and 100 mm.

The sacrificial clusters, each one consisting of nanofibers, which will compose said sacrificial hierarchical scaffold, could have an average diameter comprised between 1 and 10000 µm, in particular comprised between 50 and 200 µm.

According to an additional aspect of the invention, the number of sacrificial clusters inside said sacrificial hierarchical scaffold could be comprised between 2 and 10000, in particular comprised between 10 and 1000, for example 100.

Once obtained said sacrificial hierarchical scaffold, the process the present invention relates to according to any one of its embodiments provides that it is inserted inside a hollow container, as indicated in the previously described steps iii) or v), capable of containing said sacrificial scaffold without making it to adhere to the walls and by allowing to its terminal ends to outgo from the container itself.

According to an aspect of the invention, said container then could be filled in with a bioabsorbable or biostable and/or inert material capable of permeating within and/or incorporating said sacrificial hierarchical scaffold.

Examples of bioabsorbable or biostable and/or inert materials include materials of synthetic origin, such as polyesters, polyethers, polyurethanes, polyanhydrides, polycarbonates, polyamides, polyolefins and fluorinated polymers and copolymers thereof, materials of natural origin, for example polysaccharides, proteins, polypeptides, and copolymers thereof, and/or mixtures of these materials. Moreover, the material in case could advantageously be loaded and/or functionalized with organic and/or inorganic components apt to perform a biological function and/or to modify the physical-chemical and/or mechanical properties of the matrix.

According to a preferred embodiment of the invention, said material capable of permeating within and/or incorporating said sacrificial hierarchical scaffold is polymethyl methacrylate.

As provided in the process according to one of the previously described embodiments, said material will be left to solidify until forming a solid matrix capable of incorporating and/or integrating said sacrificial hierarchical scaffold.

According to an aspect of the invention, the removal of said sacrificial hierarchical scaffold could be performed by using a process capable of solubilizing and/or removing said sacrificial hierarchical scaffold.

Examples of solvents usable for the solubilization of said sacrificial hierarchical scaffold include aqueous and alcohol-based solvents, for example water, ethanol, methanol, isopropyl alcohol, and/or acid solvents, for example trifluoroacetic acid, formic acid, acetic acid, and/or organic solvents, for example acetone, methyl ethyl ketone, dimethylformamide, tetrahydrofuran, dimethylacetamide, dimethylsulphoxide, toluene, chloroform, dichloromethane, hexafluoroisopropanol, N-methyl-2-pyrrolidone, ethyl acetate, hexane, heptane, ethyl ether, cyclohexane, toluene, trichloroethanol.

A preferred example of solvent usable for the dissolution of said sacrificial hierarchical scaffold is distilled water.

The ends of the sacrificial electrospun scaffold, left outside the hollow container, will favour the process of interaction and solubilization and/or removal of the nanofibers by the process and/or solvent.

The porous hierarchical scaffold obtainable by means of the process the invention relates to, according to any one of the previously described embodiments, could have a length comprised between 0.1 and 2000 mm, in particular between 0.5 and 200 nm, and/or an average diameter comprised between 0.1 and 200 mm, in particular between 0.1 and 100 mm.

According to an aspect of the invention, the internal pores of said porous hierarchical scaffold, generated at the end of the step for removing/dissolving the sacrificial hierarchical scaffold, advantageously will have an average diameter comprised between 10 and 1000000 nm, in particular comprised between 5 and 1000 µm.

The present invention also relates to a hierarchical scaffold for the replacement, repair, regeneration, reconstruction and/or simulation of a bone tissue, in particular of the trabecular and/or cortical bone tissue comprising or consisting of a solid matrix characterized by internal pores organized according to a hierarchical structure, obtainable by means of the herein described process according to any one of the embodiments thereof.

According to an embodiment, said porous hierarchical scaffold and/or the nanofibers of the possible external electrospun membrane thereof it consists, could be loaded and/or functionalized with components of organic and/or inorganic nature performing a biological action and/or an action for changing the chemical-physical and/or mechanical properties of the tissue in which the scaffold could be used. For example, components of organic and/or inorganic nature which could be used are drugs, growth factors, antibacterial substances, peptides, hydroxyapatites, phosphates, bioglasses, metal oxides, graphene, carbon nanotubes.

The loading and/or functionalization procedures are known to the person skilled in the art.

The invention further relates to an implantable prosthetic device comprising a porous hierarchical scaffold according to any one of the herein described embodiments. Herein a method is also described for the regeneration or replacement of tissues, in particular the bone tissue, comprising a step of implanting in a subject requiring it, a porous hierarchical scaffold according to any one of the previously described embodiments.

The herein described scaffold could be used in an *in vitro* and/or *in vivo* method for the production of bone tissue, for example a method wherein cells are cultivated *in vitro* with the scaffold and/or implanted inside the human and/or animal body to allow the regeneration of the damaged tissue.

The herein described scaffold could further be used for the production of sensors and actuators, for example by incorporating piezoelectric materials.

The process related to the first steps of preparing the scaffold the present invention relates to are described in the International patent application WO2018229615.

### EXAMPLES

### Example 1

A prototype of hierarchical scaffold made of surgical polymethylmethacrylate (PMMA) with internal hierarchical structure soluble in pullulan having length of 20 mm (average diameter 5-10 mm) was developed.

The electrospun hierarchical structure, having length of about 100 mm, consisted of 50 axially aligned clusters, consisted of nanofibers of pullulan (average diameter of clusters of nanofibers and/or microfibers 200-400 micrometres, diameter of nanofibers 800-100 nm). Within the group of clusters, a plurality of balled-up clusters of nanofibers of pullulan were inserted (by filling up internally the group of axial clusters for a length of about 50 mm). The pullulan sheath was produced on them by electrospinning the previously described same pullulan solution. The composition is prepared with 17%(w/v) of pullulan dissolved in distilled water. The sheath was produced by electrospinning for 10 minutes, alternating periods of stasis of the group of clusters of nanofibers with periods of rotation. Spinning conditions for the production of one single cluster of nanofibers:
- spinning with 1 metal needle;
- range of syringe pump 0.9 ml/h;
- voltage of electric field 26 kV;
- rotation speed of collector 10 rpm;
- distance needle-collector about 200-250 mm;
- useful thickness of produced cluster of nanofibers 200-400 micrometres.

Once the clusters of nanofibers are cut into samples, each one having 100-mm length, they were aligned axially around a group of clusters obtained with the same procedure but balled-up to obtain micrometric pores. Once the group was fastened on a rotating machine, an external sheath of nanofibers was produced by applying the following conditions:
- spinning with 1 needle;
- range of syringe pump 0.9 ml/h;
- voltage of electric field 26 kV;
- metal collector;
- distance collector-needle/s 200-250 mm;
- distance between group of clusters and flat collector less than 5 mm;
- rotation speed of the group of clusters of nanofibers about 10-25 rpm;
- immobility periods of the group of clusters of nanofibers about 1-2 min;
- rotation periods of the group of clusters of nanofibers about 30 seconds;
- useful thickness of the sheath: 1-50 micrometres.

The structure was then inserted in an internally hollow container without making it to adhere to the walls and by allowing to its ends to outgo from the container itself. Such cavity was filled up with polymethylmethacrylate, by wholly incorporating the electrospun structure and by permeating inside thereof. Polymethylmethacrylate was left to dry up overnight. The construct was then removed from the container and dipped in distilled water until complete dissolution of the electrospun hierarchical structure.

## Claims

1. A process for the preparation of a porous hierarchical scaffold for the replacement, repair, regeneration, reconstruction and/or simulation of a bone tissue, in particular of the trabecular and/or cortical bone tissue comprising the following steps:
i) preparing by electrospinning a plurality of sacrificial clusters of nanofibers;
ii) positioning said plurality of clusters prepared in step i) so as to form at least a single group;
iii) electrospinning nanofibers so as to coat said at least one group of clusters with a sacrificial porous sheath, consisting of nanofibers, so as to form an external coating and to compact said plurality of clusters;
iv) inserting the sacrificial hierarchical scaffold obtained with step iii) inside a hollow container, leaving the terminal ends exposed;
v) filling said hollow container with a material capable of permeating within and/or incorporating said sacrificial hierarchical scaffold;
vi) allowing said material to solidify;
vii) removing said sacrificial hierarchical scaffold so as to obtain said porous hierarchical scaffold having internal pores organized according to a hierarchical structure.

2. The process according to claim 1, further comprising, after step iii) and before step iv), the following steps:
- coating by electrospinning the sacrificial hierarchical scaffold obtained with step ii) with one or more layers each one comprising a plurality of sacrificial clusters of nanofibers;
- electrospinning nanofibers so as to coat said one or more layers of clusters each one with a sacrificial porous sheath, consisting of nanofibers, so as to form an external coating and to compact said plurality of clusters of nanofibers.

3. The process according to claims 1 or 2, comprising the following steps:
i) preparing by electrospinning a plurality of sacrificial clusters of nanofibers;
ii) positioning said plurality of clusters prepared in step i) so as to form at least a single group;
iii) grasping said at least one group of clusters obtained in step ii) on a grip capable of rotating rigidly and in line thus by keeping said grasped group of clusters in a position suitable for the process of coating with sacrificial electrospun sheath;
iv) making by electrospinning a sacrificial sheath external to said at least one group of clusters grasped at step iii), in particular by controlling the rotation parameters of the grasped group of clusters, the geometrical parameters of the setup, and process parameters;
v) inserting the sacrificial hierarchical scaffold obtained with step iv) inside a hollow container, by leaving the terminal ends exposed;
vi) filling said hollow container with a material capable of permeating within and/or incorporating said sacrificial hierarchical scaffold;
vii) allowing said material to solidify;
viii) removing said sacrificial hierarchical scaffold so as to produce said porous hierarchical scaffold having internal pores organized according to a hierarchical structure.

4. The process according to any one of claims 1 to 3, comprising the following steps:
(i) preparing by electrospinning a plurality of sacrificial clusters of nanofibers;
(ii) positioning said plurality of clusters prepared in step i) so as to form a plurality of groups of clusters;
(iii) grasping each group of clusters obtained in step ii) on a grip capable of rotating rigidly and in line thus by keeping each grasped group of clusters in a position suitable for the process of coating with sacrificial electrospun sheath;
(iv) making by electrospinning a sacrificial sheath external to each group of clusters grasped at step iii), in particular by controlling the rotation parameters of the grasped group of clusters, the geometrical parameters of the setup, and process parameters;
(v) electrospinning nanofibers so as to coat said plurality of groups of clusters with a sacrificial porous sheath, consisting of nanofibers, so as to form an external coating and to compact said plurality of groups of clusters;
(vi) inserting the sacrificial hierarchical scaffold obtained with step v) inside a hollow container, by leaving the terminal ends exposed;
(vii) filling said hollow container with a material capable of permeating within and/or incorporating said sacrificial hierarchical scaffold;
(viii) allowing said material to solidify;
(ix) removing said sacrificial hierarchical scaffold so as to produce said porous hierarchical scaffold having internal pores organized according to a hierarchical structure.

5. The process according to claims 1 to 4, comprising an additional step of:
- electrospinning nanofibers so as to coat said porous hierarchical scaffold obtained in the final step with a membrane consisting of nanofibers, so as to form an external coating, preferably wherein said membrane consisting of nanofibers is prepared by electrospinning a solution of bioabsorbable or biostable and/or inert material of synthetic or natural origin, in particular a solution of polylactic acid and/or a mixture of polylactic acid and collagen.

6. The process according to any one of claims 1 to 5, wherein said sacrificial clusters, each one consisting of nanofibers, are positioned and/or connected to each other according to an axial alignment with respect to the sacrificial hierarchical scaffold, and/or according to a circumferential orientation with respect to the sacrificial hierarchical scaffold, and/or according to a radial orientation with respect to the sacrificial hierarchical scaffold, and/or randomly, and/or according to combinations thereof, preferably wherein said sacrificial clusters, each one consisting of nanofibers, are positioned between each other according to an axial alignment with respect to the axis of the sacrificial hierarchical scaffold so as to form a central group of compact sacrificial clusters.

7. The process according to any one of claims 1 to 6, wherein said sacrificial clusters and said porous sacrificial sheath/s are made of a material selected from polyesters, polyethers, polyurethanes, polyanhydrides, polycarbonates, polyamides, polyolefins, fluorinated polymers, polysaccharides, proteins, polypeptides and copolymers and/or mixtures thereof, preferably wherein said sacrificial clusters and said sacrificial sheaths are prepared by electrospinning a solution of pullulan and/or polyethylene oxide.

8. The process according to any one of claims 1 to 7, wherein during step iii) or v) said sacrificial hierarchical scaffold is inserted inside said hollow container without adhering to the walls of said container.

9. The process according to any one of claims 1 to 8, wherein said material capable of permeating within and/or incorporating said sacrificial hierarchical scaffold is a bioabsorbable or biostable and/or inert material selected from polyesters, polyethers, polyurethanes, polyanhydrides, polycarbonates, polyamides, polyolefins, fluorinated polymers, acrylates, polysaccharides, proteins, polypeptides and copolymers and/or mixtures thereof, preferably is polymethylmethacrylate.

10. The process according to any one of claims 1 a 9, wherein said removal of said sacrificial hierarchical scaffold is carried out by using a process capable of eliminating said sacrificial hierarchical scaffold by means of a solvent, preferably wherein said solvent is selected from aqueous and alcohol-based solvents, for example water, ethanol, methanol, isopropyl alcohol, and/or acid solvents, for example trifluoroacetic acid, formic acid, acetic acid, and/or organic solvents, for example acetone, methyl ethyl ketone, dimethylformamide, tetrahydrofuran, dimethylacetamide, dimethylsulphoxide, toluene, chloroform, dichloromethane, hexafluoroisopropanol, N-methyl-2-pyrrolidone, ethyl acetate, hexane, heptane, ethyl ether, cyclohexane, trichloroethanol.

11. The process according to any one of claims 1 to 10, wherein said porous hierarchical scaffold has a length between 0.1 and 2000 mm, in particular between 0.5 and 200 nm, and/or an average diameter comprised between 0.1 and 200 mm, in particular between 0.1 and 100 mm.

12. The porous hierarchical scaffold for the replacement, repair, regeneration, reconstruction and/or simulation of a bone tissue, in particular of the trabecular and/or cortical bone tissue obtainable by a process according to any one of claims 1 to 11.

13. The scaffold according to claim 12, wherein said material capable of permeating within and/or incorporating said sacrificial hierarchical scaffold and/or said external membrane are loaded and/or functionalized with organic and/or inorganic components apt to perform a biological action and/or change in the chemical-physical and/or mechanical properties of said tissue, preferably selected from drugs, growth factors, antibacterial compounds, peptides, hydroxyapatites, phosphates, bioglasses, metal oxides, graphene, carbon nanotubes or mixtures thereof.

14. An implantable prosthetic device comprising a scaffold according to any one of claims 12 or 13.

## Patentansprüche

1. Verfahren für die Herstellung eines porösen hierarchischen Gerüsts für den Ersatz, die Reparatur, die Regeneration, die Rekonstruktion und/oder die Simulation eines Knochengewebes, insbesondere des trabekulären und/oder kortikalen Knochengewebes, umfassend die folgenden Schritte:
i) Herstellen einer Vielzahl von Opferclustern von Nanofasern durch Elektrospinnen;
ii) Positionieren der Vielzahl von Clustern, die in Schritt i) hergestellt wird, um mindestens eine einzige Gruppe auszubilden;
iii) Elektrospinnen von Nanofasern, um die mindestens eine Gruppe von Clustern mit einer porösen Opferhülle, die aus Nanofasern besteht, zu beschichten, um eine äußere Beschichtung auszubilden und um die Vielzahl von Clustern zu verdichten;
iv) Einsetzen des hierarchischen Opfergerüsts, das in Schritt iii) erhalten wird, in das Innere eines hohlen Behälters, was die terminalen Enden freigelegt lässt;
v) Füllen des hohlen Behälters mit einem Material, das in der Lage ist, innerhalb das hierarchische Opfergerüst einzudringen und/oder dieses zu integrieren;
vi) Erlauben dem Material, sich zu verfestigen;
vii) Entfernen des hierarchischen Opfergerüsts, um das poröse hierarchische Gerüst zu erhalten, das innere Poren aufweist, die gemäß einer hierarchischen Struktur organisiert sind.

2. Verfahren nach Anspruch 1, ferner umfassend, nach Schritt iii) und vor Schritt iv), die folgenden Schritte:
- Beschichten durch Elektrospinnen des hierarchischen Opfergerüsts, das in Schritt ii) erhalten wird, mit einer oder mehreren Schichten, jeweils umfassend eine Vielzahl von Opferclustern von Nanofasern;
- Elektrospinnen von Nanofasern, um die eine oder die mehreren Schichten von Clustern jeweils mit einer porösen Opferhülle, die aus Nanofasern besteht, zu beschichten, um eine äußere Beschichtung auszubilden und um die Vielzahl von Clustern von Nanofasern zu verdichten.

3. Verfahren nach Ansprüchen 1 oder 2, umfassend die folgenden Schritte:
i) Herstellen einer Vielzahl von Opferclustern von Nanofasern durch Elektrospinnen;
ii) Positionieren der Vielzahl von Clustern, die in Schritt i) hergestellt wird, um mindestens eine einzige Gruppe auszubilden;
iii) Erfassen der mindestens einen Gruppe von Clustern, die in Schritt ii) erhalten wird, auf einen Griff, der in der Lage ist, sich starr und linear zu drehen, somit durch Halten der erfassten Gruppe von Clustern in einer Position, die für den Prozess des Beschichtens mit der elektrogesponnenen Opferhülle geeignet ist;
iv) Anfertigen einer Opferhülle außerhalb der mindestens einen Gruppe von Clustern, die in Schritt iii) erfasst wird, durch Elektrospinnen, insbesondere durch Steuern der Drehparameter der erfassten Gruppe von Clustern, der geometrischen Parameter des Aufbaus und der Prozessparameter;
v) Einsetzen des hierarchischen Opfergerüsts, das in Schritt iv) erhalten wird, in das Innere eines hohlen Behälters dadurch, dass die terminalen Enden freigelegt gelassen werden;
vi) Füllen des hohlen Behälters mit einem Material, das in der Lage ist, innerhalb des hierarchischen Opfergerüsts einzudringen und/oder dieses zu integrieren;
vii) Erlauben dem Material, sich zu verfestigen;
viii) Entfernen des hierarchischen Opfergerüsts, um das poröse hierarchische Gerüst zu produzieren, das innere Poren aufweist, die gemäß einer hierarchischen Struktur organisiert sind.

4. Verfahren nach einem der Ansprüche 1 bis 3, umfassend die folgenden Schritte:
(i) Herstellen einer Vielzahl von Opferclustern von Nanofasern durch Elektrospinnen;
(ii) Positionieren der Vielzahl von Clustern, die in Schritt i) hergestellt wurden, um eine Vielzahl von Gruppen von Clustern auszubilden;
(iii) Erfassen jeder Gruppe von Clustern, die in Schritt ii) erhalten wird, auf einem Griff, der in der Lage ist, sich starr und linear zu drehen, somit durch Halten jeder erfassten Gruppe von Clustern in einer Position, die für den Prozess des Beschichtens mit der elektrogesponnenen Opferhülle geeignet ist;
(iv) Anfertigen einer Opferhülle außerhalb jeder Gruppe von Clustern, die in Schritt iii) erfasst wird, durch Elektrospinnen, insbesondere durch Steuern der Drehparameter der erfassten Gruppe von Clustern, der geometrischen Parameter des Aufbaus und der Prozessparameter;
(v) Elektrospinnen von Nanofasern, um die Vielzahl von Gruppen von Clustern mit einer porösen Opferhülle, die aus Nanofasern besteht, zu beschichten, um eine äußere Beschichtung auszubilden und um die Vielzahl von Gruppen von Clustern zu verdichten;
(vi) Einsetzen des hierarchischen Opfergerüsts, das in Schritt v) erhalten wird, in das Innere eines hohlen Behälters dadurch, dass die terminalen Enden freigelegt gelassen werden;
(vii) Füllen des hohlen Behälters mit einem Material, das in der Lage ist, in das hierarchische Opfergerüst einzudringen und/oder dieses zu integrieren;
(viii) Erlauben dem Material, sich zu verfestigen;
(ix) Entfernen des hierarchischen Opfergerüsts, um das poröse hierarchische Gerüst zu produzieren, das innere Poren aufweist, die gemäß einer hierarchischen Struktur organisiert sind.

5. Verfahren nach den Ansprüchen 1 bis 4, umfassend einen zusätzlichen Schritt:
- Elektrospinnen von Nanofasern, um das poröse hierarchische Gerüst, das in dem letzten Schritt erhalten wird, mit einer Membran, die aus Nanofasern besteht, zu beschichten, um eine äußere Beschichtung auszubilden, vorzugsweise wobei die Membran, die aus Nanofasern besteht, durch Elektrospinnen einer Lösung aus bioabsorbierbarem oder biostabilem und/oder inertem Material synthetischen oder natürlichen Ursprungs, insbesondere einer Lösung aus Polymilchsäure und/oder einer Mischung aus Polymilchsäure und Collagen, hergestellt wird.

6. Verfahren nach einem der Ansprüche 1 bis 5, wobei die Opfercluster, die jeweils aus Nanofasern bestehen, gemäß einer axialen Ausrichtung in Bezug auf das hierarchische Opfergerüst und/oder gemäß einer Umfangsorientierung in Bezug auf das hierarchische Opfergerüst und/oder gemäß einer radialen Orientierung in Bezug auf das hierarchische Opfergerüst und/oder zufällig und/oder gemäß Kombinationen davon positioniert und/oder miteinander verbunden werden, vorzugsweise wobei die Opfercluster, die jeweils aus Nanofasern bestehen, gemäß einer axialen Ausrichtung in Bezug auf die Achse des hierarchischen Opfergerüsts zwischen einander positioniert werden, um eine zentrale Gruppe von kompakten Opferclustern auszubilden.

7. Verfahren nach einem der Ansprüche 1 bis 6, wobei die Opfercluster und die poröse(n) Opferhülle(n) aus einem Material angefertigt sind, das aus Polyestern, Polyethern, Polyurethanen, Polyanhydriden, Polycarbonaten, Polyamiden, Polyolefinen, fluorierten Polymeren, Polysacchariden, Proteinen, Polypeptiden und Copolymeren und/oder Mischungen davon ausgewählt ist, vorzugsweise wobei die Opfercluster und die Opferhüllen durch Elektrospinnen einer Lösung aus Pullulan und/oder Polyethylenoxid hergestellt werden.

8. Verfahren nach einem der Ansprüche 1 bis 7, wobei während Schritt iii) oder v) das hierarchische Opfergerüst in das Innere des hohlen Behälters eingesetzt wird, ohne an den Wänden des Behälters zu haften.

9. Verfahren nach einem der Ansprüche 1 bis 8, wobei das Material, das in der Lage ist, innerhalb des hierarchischen Opfergerüsts einzudringen und/oder dieses zu integrieren, ein bioabsorbierbares oder biologisch stabiles und/oder inertes Material ist, das aus Polyestern, Polyethern, Polyurethanen, Polyanhydriden, Polycarbonaten, Polyamiden, Polyolefinen, fluorierten Polymeren, Acrylaten, Polysacchariden, Proteinen, Polypeptiden und Copolymeren und/oder Mischungen davon ausgewählt ist, vorzugsweise Polymethylmethacrylat ist.

10. Verfahren nach einem der Ansprüche 1 a 9, wobei die Entfernung des hierarchischen Opfergerüsts durch Verwenden eines Verfahrens vorgenommen wird, das in der Lage ist, das hierarchische Opfergerüst mittels eines Lösungsmittels zu entfernen, vorzugsweise wobei das Lösungsmittel aus wässrigen und alkoholbasierten Lösungsmitteln, beispielsweise Wasser, Ethanol, Methanol, Isopropylalkohol, und/oder sauren Lösungsmitteln, beispielsweise Trifluoressigsäure, Ameisensäure, Essigsäure, und/oder organischen Lösungsmitteln, beispielsweise Aceton, Methylethylketon, Dimethylformamid, Tetrahydrofuran, Dimethylacetamid, Dimethylsulfoxid, Toluol, Chloroform, Dichlormethan, Hexafluorisopropanol, N-Methyl-2-pyrrolidon, Ethylacetat, Hexan, Heptan, Ethylether, Cyclohexan, Trichlorethanol ausgewählt ist.

11. Verfahren nach einem der Ansprüche 1 bis 10, wobei das poröse hierarchische Gerüst eine Länge zwischen 0,1 und 2000 mm, insbesondere zwischen 0,5 und 200 nm, und/oder einen durchschnittlichen Durchmesser, der zwischen 0,1 und 200 mm, insbesondere zwischen 0,1 und 100 mm, liegt, aufweist.

12. Poröses hierarchisches Gerüst für den Ersatz, die Reparatur, die Regeneration, die Rekonstruktion und/oder die Simulation eines Knochengewebes, insbesondere des trabekulären und/oder kortikalen Knochengewebes, das durch ein Verfahren nach einem der Ansprüche 1 bis 11 erhältlich ist.

13. Gerüst nach Anspruch 12, wobei das Material, das in der Lage ist, innerhalb des hierarchischen Opfergerüsts und/oder der äußeren Membran einzudringen und/oder diese(s) zu integrieren, mit organischen und/oder anorganischen Komponenten beladen und/oder funktionalisiert sind, die eine biologische Wirkung und/oder Veränderung der chemisch-physikalischen und/oder mechanischen Eigenschaften des Gewebes durchführen können, die vorzugsweise aus Arzneimitteln, Wachstumsfaktoren, antibakteriellen Verbindungen, Peptiden, Hydroxyapatiten, Phosphaten, Biogläsern, Metalloxiden, Graphen, Kohlenstoffnanoröhrchen oder Mischungen davon ausgewählt sind.

14. Implantierbare Prothesenvorrichtung, umfassend ein Gerüst nach einem der Ansprüche 12 oder 13.

## Revendications

1. Procédé de préparation d'un échafaudage poreux hiérarchique pour le remplacement, la réparation, la régénération, la reconstruction et/ou la simulation d'un tissu osseux, en particulier du tissu osseux trabéculaire et/ou cortical, comprenant les étapes suivantes :
i) la préparation par électrofilage une pluralité d'amas sacrificiels de nanofibres ;
ii) le positionnement de ladite pluralité de grappes préparées à l'étape i) de manière à former au moins un seul groupe ;
iii) l'électrofilage de nanofibres afin de recouvrir ledit au moins un groupe de grappes d'une gaine poreuse sacrificielle, constituée de nanofibres, de manière à former un revêtement externe et à compacter ladite pluralité de grappes ;
iv) l'insertion de l'échafaudage hiérarchique sacrificiel obtenu à l'étape iii) à l'intérieur d'un récipient creux, en laissant les extrémités terminales exposées ;
v) le remplissage dudit récipient creux d'un matériau capable de pénétrer à l'intérieur et/ou d'incorporer ledit échafaudage hiérarchique sacrificiel ;
vi) la permission de ladite matière à solidifier ;
vii) le retirait dudit échafaudage hiérarchique sacrificiel afin d'obtenir ledit échafaudage hiérarchique poreux dont les pores internes sont organisés selon une structure hiérarchique.

2. Procédé selon la revendication 1, comprenant en outre, après l'étape iii) et avant l'étape iv), les étapes suivantes :
- le revêtement par électrofilage de l'échafaudage hiérarchique sacrificiel obtenu à l'étape ii) d'une ou de plusieurs couches comprenant chacune une pluralité d'amas sacrificiels de nanofibres ;
- l'électrofilage de nanofibres de manière à revêtir ladite ou plusieurs couches d'amas chacune d'une gaine poreuse sacrificielle, constituée de nanofibres, de manière à former un revêtement externe et à compacter ladite pluralité d'amas de nanofibres.

3. Procédé selon les revendications 1 ou 2, comprenant en outre les étapes suivantes :
i) la préparation par électrofilage une pluralité d'amas sacrificiels de nanofibres ;
ii) le positionnement de ladite pluralité de grappes préparées à l'étape i) de manière à former au moins un seul groupe ;
iii) le saisissement dudit au moins un groupe de grappes obtenu à l'étape ii) sur une pince capable de tourner de façon rigide et en ligne en maintenant ainsi ledit groupe de grappes saisi dans une position appropriée pour le processus d'enrobage avec une gaine sacrificielle électrospun ;
iv) la fabrication par électrofilage une gaine sacrificielle externe audit au moins un groupe de grappes saisi à l'étape iii), notamment en commandant les paramètres de rotation du groupe de grappes saisi, les paramètres géométriques de l'installation et les paramètres du procédé ;
v) l'insertion de l'échafaudage hiérarchique sacrificiel obtenu à l'étape iv) à l'intérieur d'un récipient creux, en laissant les extrémités terminales exposées ;
vi) le remplissage dudit récipient creux d'un matériau capable de pénétrer à l'intérieur de l'échafaudage hiérarchique sacrificiel et/ou de l'incorporer ;
vii) la solidification du matériau ;
viii) le retrait dudit échafaudage hiérarchique sacrificiel de manière à produire ledit échafaudage hiérarchique poreux dont les pores internes sont organisés selon une structure hiérarchique.

4. Procédé selon l'une quelconque des revendications 1 à 3, comprenant les étapes suivantes :
(i) la préparation par électrofilage d'une pluralité d'amas sacrificiels de nanofibres ;
(ii) le positionnement de ladite pluralité de grappes préparées à l'étape i) de manière à former une pluralité de groupes de grappes ;
(iii) le saisissement de chaque groupe de grappes obtenu à l'étape ii) sur une pince capable de tourner de façon rigide et en ligne en maintenant ainsi chaque groupe de grappes saisi dans une position appropriée pour le processus d'enrobage avec une gaine sacrificielle électrofilée ;
(iv) la fabrication par électrofilage d'une gaine sacrificielle externe à chaque groupe de grappes saisi à l'étape iii), notamment en contrôlant les paramètres de rotation du groupe de grappes saisi, les paramètres géométriques de l'installation et les paramètres du processus ;
(v) l'électrofilage de nanofibres de manière à recouvrir ladite pluralité de groupes de grappes d'une gaine poreuse sacrificielle, constituée de nanofibres, de manière à former un revêtement externe et à compacter ladite pluralité de groupes de grappes ;
(vi) l'insertion de l'échafaudage hiérarchique sacrificiel obtenu à l'étape v) dans un récipient creux, en laissant les extrémités exposées ;
(vii) le remplissage dudit récipient creux d'un matériau capable de pénétrer dans et/ou d'incorporer ledit échafaudage hiérarchique sacrificiel ;
(viii) la permission à ladite matière de se solidifier ;
(ix) le retrait dudit échafaudage hiérarchique sacrificiel de manière à produire ledit échafaudage hiérarchique poreux dont les pores internes sont organisés selon une structure hiérarchique.

5. Procédé selon l'une quelconque des revendications 1 à 4, comprenant une étape supplémentaire consistant à :
- l'électrofilage de nanofibres afin de recouvrir ledit échafaudage poreux hiérarchique obtenu à l'étape finale d'une membrane constituée de nanofibres, de manière à former un revêtement externe, de préférence dans lequel ladite membrane constituée de nanofibres est préparée par électrofilage d'une solution de matériau bioabsorbable ou biostable et/ou inerte d'origine synthétique ou naturelle, en particulier d'une solution d'acide polylactique et/ou d'un mélange d'acide polylactique et de collagène.

6. Procédé selon l'une quelconque des revendications 1 à 5, dans lequel lesdits amas sacrificiels, chacun constitué de nanofibres, sont positionnés et/ou connectés les uns aux autres selon un alignement axial par rapport à l'échafaudage hiérarchique sacrificiel, et/ou selon une orientation circonférentielle par rapport à l'échafaudage hiérarchique sacrificiel, et/ou selon une orientation radiale par rapport à l'échafaudage hiérarchique sacrificiel, et/ou de manière aléatoire, et/ou selon des combinaisons de celles-ci, et/ou selon une orientation radiale par rapport à l'échafaudage hiérarchique sacrificiel, et/ou de manière aléatoire, et/ou selon des combinaisons de celles-ci, de préférence dans lesquelles lesdites grappes sacrificielles, chacune constituée de nanofibres, sont positionnées entre elles selon un alignement axial par rapport à l'axe de l'échafaudage hiérarchique sacrificiel de manière à former un groupe central de grappes sacrificielles compactes.

7. Procédé selon l'une quelconque des revendications 1 à 6, dans lequel lesdits amas sacrificiels et lesdites gaines sacrificielles poreuses sont constitués d'un matériau choisi parmi les polyesters, les polyéthers, les polyuréthanes, les polyanhydrides, les polycarbonates, les polyamides, les polyoléfines, les polymères fluorés, les polysaccharides, les protéines, les polypeptides et les copolymères et/ou leurs mélanges, de préférence dans lequel lesdits amas sacrificiels et lesdites gaines sacrificielles sont préparés par électrofilage d'une solution de pullulan et/ou d'oxyde de polyéthylène.

8. Procédé selon l'une quelconque des revendications 1 à 7, dans lequel, au cours de l'étape iii) ou v), ledit échafaudage hiérarchique sacrificiel est inséré à l'intérieur dudit récipient creux sans adhérer aux parois dudit récipient.

9. Procédé selon l'une quelconque des revendications 1 à 8, dans lequel ledit matériau capable de pénétrer dans et/ou d'incorporer ledit échafaudage hiérarchique sacrificiel est un matériau bioabsorbable ou biostable et/ou inerte choisi parmi les polyesters, les polyéthers, les polyuréthanes, les polyanhydrides, les polycarbonates, les polyamides, les polyoléfines, les polymères fluorés, les acrylates, les polysaccharides, les protéines, les polypeptides et les copolymères et/ou mélanges de ceux-ci, de préférence le polyméthacrylate de méthyle.

10. Procédé selon l'une quelconque des revendications 1 à 9, dans lequel l'élimination dudit échafaudage hiérarchique sacrificiel est effectuée en utilisant un procédé capable d'éliminer ledit échafaudage hiérarchique sacrificiel au moyen d'un solvant, de préférence dans lequel ledit solvant est choisi parmi les solvants aqueux et les solvants à base d'alcool, par exemple l'eau, l'éthanol, le méthanol, l'alcool isopropylique, et/ou des solvants acides, par exemple l'acide trifluoroacétique, l'acide formique, l'acide acétique, et/ou des solvants organiques, par exemple l'acétone, la méthyléthylcétone, le diméthylformamide, le tétrahydrofurane, le diméthylacétamide, le diméthylacétamide, le diméthylacétamide, le tétrahydrofurane, etc, diméthylacétamide, diméthylsulfoxyde, toluène, chloroforme, dichlorométhane, hexafluoroisopropanol, N-méthyl-2-pyrrolidone, acétate d'éthyle, hexane, heptane, éther éthylique, cyclohexane, trichloréthanol.

11. Procédé selon l'une quelconque des revendications 1 à 10, dans lequel ledit échafaudage hiérarchique poreux a une longueur comprise entre 0,1 et 2000 mm, en particulier entre 0,5 et 200 nm, et/ou un diamètre moyen compris entre 0,1 et 200 mm, en particulier entre 0,1 et 100 mm.

12. Échafaudage poreux hiérarchique pour le remplacement, la réparation, la régénération, la reconstruction et/ou la simulation d'un tissu osseux, en particulier du tissu osseux trabéculaire et/ou cortical, obtenu par un procédé selon l'une quelconque des revendications 1 à 11.

13. Échafaudage selon la revendication 12, dans lequel lesdits matériaux capables de pénétrer dans et/ou d'incorporer ledit échafaudage hiérarchique sacrificiel et/ou ladite membrane externe sont chargés et/ou fonctionnalisés avec des composants organiques et/ou inorganiques aptes à exercer une action biologique et/ou à modifier les propriétés chimiques-physiques et/ou mécaniques dudit tissu, de préférence choisis parmi les médicaments, les facteurs de croissance, les composés antibactériens, les peptides, les hydroxyapatites, les phosphates, les bioglasses, les oxydes métalliques, le graphène, les nanotubes de carbone ou les mélanges de ces derniers.

14. Dispositif prothétique implantable comprenant un échafaudage selon l'une quelconque des revendications 12 ou 13.
